# EUROPEAN PATENT SPECIFICATION

(11) **EP 2 809 260 B1**
(45) Date of publication and mention of the grant of the patent: **20.07.2016**
(21) Application number: 12818556.8
(22) Date of filing: 08.11.2012
(51) Int. Cl.: A61F 2/00

(54) **Medical device for the reconstruction of parastomal hernias and/or for the prevention of their development**
Medizinische Vorrichtung zur Rekonstruktion parastomaler Hernien und/oder zur Verhinderung von deren Entwicklung
Dispositif médical de reconstruction d'hernies parastomales et/ou de prévention de leur développement

(30) Priority: 31.01.2012 HU P1200068
(43) Date of publication of application: 10.12.2014
(73) Proprietor: Replant Cardo KFT., 1119 Budapest (HU)
(72) Inventor: NAGY, Attila, H-8200 Veszprém (HU)
(74) Representative: Kovari, György
(86) International application number: PCT/HU2012/000120
(87) International publication number: WO 2013/114145

(56) References cited:
- US-A- 4 854 316
- US-A1- 2011 251 452
- US-B1- 6 632 450

## Description

The subject of the invention relates to a medical device used for the reconstruction of parastomal hernias and or for the prevention of their development.

In the case of certain illnesses, primarily cancer of the large intestine a loop of the large intestine is led out through the abdominal wall in the course of a surgical intervention and then the stoma located on the abdominal wall is fixed to the skin with stitches around it. A removable and replaceable ostomy device may be attached to the abdominal wall which seals off the intestinal stoma from the environment and which retains the faeces in a known way, the secure positioning and wearability of which has a decisive effect on the resocialisation of the patient.

Partly for anatomical reasons and partly as a consequence of errors during the operation in some 15-30% of cases a hernia - so called parastomal hernia develops around the stoma located on the abdominal wall, a proportion of which, approx. 20-50% are not only problematic due to a change in the shape of the body of the patient, but they may, on the one hand, cause an intestinal obstruction and, on the other hand, make it impossible to wear the aforementioned ostomy device or to secure it safely, which - as we have already mentioned - may have an exceedingly disadvantageous effect on the resocialisation of the patient.

Several types of surgical method are known of for the correction of parastomal hernias - e.g. local reconstruction of the hernia with resection of the hernial sac and reconstruction of the abdominal wall or annulorrhaphy, relocation of the stoma, insertion of abdominal wall replacement materials, etc. - however, none of these have proven suitable in themselves to be reassuring solutions to the problem. Hernia relapse is frequent, the proportion of septic and other complications following surgery is high (Londono-Schimmer EE, Leong Ap, Phillips RK, Life table analysis of stomal complications following colostomy, Diseases of the colon and rectum, 37; 916-920, 1994; Makela JT, Turku PH, Laitinen ST, Analysis of late stomal complications following ostomy surgery, Annales chirurgiae et gynaecologiae, 86; 305-310, 1997; Oriz H, Sara MJ, Armendariz P, de Miguel M, Marti J, Chocarro C., Does the frequency of paracolostomy hernias depend on the position of the colostomy in the abdominal wall? International journal of colorectal disease, 9; 65-67, 1994; Rubin MS, Schoetz DJ Jr, Matthews JB, Parastomal hernia. Is stoma relocation superior to fascial repair? Archives of Surgery, 129; 413-418, 1994). Kasparek R., Willis S., Klinge U. Schumpelik V. Update on incisional hernia Parastomal hernia Chirurg 73. 895-8 2002. A large percentage of the foreign materials used for implantation in the past were rejected, afterwards frequently leaving a larger hernia than what they were used to treat (Botet X, Boldo E, Llaurando JM, Colonic parastomal hernia repair by translocation without formal laparotomy, British Journal of Surgery 83; 981, 1996; Morris-Stiff G'Hughes LE, The continuing challenge of parastomal hernia: failure of a novel polypropylene mesh repair, Annals of the Royal College of Surgeins of England, 80;184-187,1998. Aldridge A J, Simson J N Erosion and perforation of colon by synthetic mesh in a recurrent paracolostomy hernia Hernia 5. 110-2. 2001.

In the field of the development of living tissue-friendly plastic sheets and plates, especially plastic meshes and woven plastic textile plates and plastic meshes new result achieved recently have made it possible to attempt to use these in potentially septic environments as well. According to the experience gained in the recent past polypropylene-based and the "Goretex" brand-named abdominal wall replacement plates and meshes are fundamentally reliable from the point of view of compatibility with human tissue, their rejection from the body, as compared to the mesh materials used in the past, is now a rare occurrence. Several companies producing suture thread and other medical auxiliary materials distribute devices suitable for implantation, e.g. "Bard", "Braun Mellsungen", "Johnson and Johnson", etc. The significant advantage of the aforementioned "Goretex" material is that they can be placed in the abdominal cavity to be in direct contact with the peritoneal covering of the intra-abdominal organs without the risk of intra-abdominal adhesions in the vicinity.

Several methods are known of to locate plastic meshes around parastomal hernias. The disadvantage of single-layer meshes placed on the fascia or on the internal surface above the peritoneum of the abdominal wall is that their edge comes into contact with the wall of the intestine that is led through them, and depending on the rigidity of the plastic material as a consequence of the motion of the abdominal wall, and the tensing of the muscles in various directions they can even cut into the wall of the intestine, and causing a faecal fistula they may destroy the otherwise mechanical support result. A mesh placed on the fascia is also questionable from the point of view of the safety of the closing of the hernia.

It is known that in the case of operations aimed at the reconstruction of parastomal hernias two plastic meshes are positioned in two layers with a gap between one another (Byers JM., Steinberg JB., Postier RG, Repair of parastomal hernias using polypropylene mesh, Archives of Surgery, 127; 1246-1347, 1992), which, however, does not stop the risk of the sawing of the plastic meshes into the wall of the intestine, at the most it only moderates it as a result of the intestine being tensioned in the area between the two plastic meshes.

According to another known solution (DeRuiter P., Bijnen AB, Successful local repair of paracolostomy hernia with a newly developed prosthetic device, International Journal of Colorectal Disease, 7; 132-140, 1992, Comment in: Int J Colorectal Dis: 8, 179, 1993) one layer of plastic mesh is used, but a small cylindrical tube member built into the mesh surrounds the stoma running through the layers of the abdominal wall. This tube member lying on the wall of the intestine at a given length - if this tube is made from a suitable material with a feature permitting it to be built into the abdominal wall, e.g. polypropylene - may be able to prevent a piece of the omentum or another small bowel loop from slipping in between the intestine and the mesh but it does not represent a perfect solution, and it does not provide space for a loop of artery to provide the blood supply for the stoma brought outside of the abdominal wall, what is more, due to scarring it may dangerously press it down, which may lead to the stoma dying or, in a milder case, to stricture. We note that to date none of the procedures presented above have been used to strengthen the vicinity of the pulled-through stoma for the purpose of preventing hernia.

It is only in inguinal hernia operations that auxiliary devices consisting of two-layered, tissue-friendly mesh sheets are used, in which the two mesh sheets are connected to each other via a distance-maintaining tube element one end of which is closed by the one sheet and the other end opens out to the surface of the other sheet. This auxiliary device is a product of the Johnson and Johnson Company (Somerville, USA). Hungarian patent application number P 01 02060 presents an auxiliary device that can be used for the reconstruction of parastomal hernias, which has plastic sheets compatible with living tissue placed in the abdominal wall. They run at a distance from one another and are connected to one another via a connecting-separating element. The connecting-separating element is formed by a tube member open at both ends which forms a channel, and which makes it possible to lead the large intestine from the abdominal cavity outside the abdominal wall. On the internal side of the channel there is a longitudinal groove that serves to accommodate the arterial loop that provides the blood supply to the bowel loop used for the stoma. At least the tube member, but favourably the sheets are from plastic mesh or from woven plastic textile.

In the case of the solution forming the subject of the Hungarian patent application detailed above, it is a problem that the longitudinal groove formed in the side wall of the aforementioned tube member - channel - accommodating the mesentary containing the arteries providing the blood supply to the stoma to be pulled through may cause the omentum or bowel loops to fall forwards or slip out early following the operation. It is also a problem that the internal surface of the tube member made from plastic mesh and binding the sheets, which comes into contact with the surface of the stoma, may cause scarred adhesion between the wall of the intestine and the material of the mesh, which adhesion my be so strong that on occasion it may even penetrate to the inner layers of the wall of the intestine. Over and above the fact that the serious scarring may restrict the wave-like motion of the intestine promoting the discharging of the content of the intestine, and that it may also involve the risk of the development of bowel perforation, in the case of reoperation it may have a disadvantageous effect on the separation of the tissues without causing them injury.

Finally, in the case of this solution it is also a problem that the external and internal plastic mesh sheets running at a distance from one another occasionally twist the cylindrical tube member connecting them around its longitudinal axis, and the intestine follows this twisting movement, and if finally the device and the intestine become fixed - even if in a slightly twisted position - this may cause a smaller or larger degree of intestinal stenosis.

The latter problem is partly overcome by the device proposed in US 4,854,316 where the parallel sheets and the connecting tubular element there between in sutured to the neighbouring abdominal portion. However, displacement and torsion of the sheets with respect to each other cannot be fully inhibited in this way since working of the muscle layers of the abdominal wall results in multidirectional movements, thus the inner and outer sheets may move independently from each other.

The task to be solved with the invention is to provide a device that promotes the prevention of parastomal hernias that overcomes the deficiencies of the known, similar solutions, i.e. of the device detailed above, and makes it possible to perform the perfect reconstruction of parastomal hernias and/or prevent their development, or at least reduce the risk of their development to the minimum.

The invention is based on the following recognitions:
The twisting of the plastic mesh tube connecting the external and internal sheets of the device according to the known Hungarian patent specification discussed above along its longitudinal axis and with this the twisting of the intestine itself as well as its fixing in a twisted position is caused by the everyday, multidirectional movement of the muscle layers of the abdominal wall, which obviously widens the intestine opening. In connection with this we make reference to an examination performed on cadavers directly after death (Rath AM, Zhang J, Chevrel JP: The sheath of the rectus abdominis muscle: an anatomical and biomechanical study. Hernia 1997; 1 (3): 139-142), according to which the rectus muscle fascias were subject to biomechanical tests in various locations - this is the muscle through which the large intestine is led to the outer surface of the abdominal wall - as was the transverse tendinous thickening of the linea arcuata running in the lower 2/3 of the rectus muscle.

According to their results resistance of the outer fascia in its part under the linea arcuata to a pulling force is greater than in its part above the linea arcuata, at the same time the other measured parameter, resistance to internal pressure, is precisely the other way round, it is greater above the linea arcuata than below it in the outer fascia and greater everywhere than in the inner fascia.

These same indicators in the inner fascia are such that the pulling force is more or less the same in the whole length of the fascia, while resistance to internal pressure is the lowest precisely in the vicinity of the linea arcuata, in other words in the region where the stoma is usually fixed, in the left lower abdominal quadrant.

This resistance to internal pressure is actually the pressure of the abdominal cavity, therefore all the conditions that cause an increase in this gradually endanger the inner fascia and the pulling force, which in life is the contractions of the rectus muscle, causes a greater effect in the outer fascia.

From the above we recognised that when creating a stoma, which is performed using the device according to the invention, and which in practice demands the formation of an opening passing through the intact muscle held between the two sheets of the device, the fixing of these muscle layers separately to each other cannot be realised. As, however, the two plastic mesh sheets of the device encompass both the front and the inner fascia of the rectus muscle, the position of the latter, ensuring it is immobile can be solved with sutures passing through the muscle layers supporting the abdominal wall. With sutures at, at least, four symmetrical points we can ensure that the vicinity of the stoma and the mesh move together and overcome the risk or possibility of twisting in the opposite direction. As a result of the fixing the possibility of the pulling apart of the edges of the opening formed in the abdominal wall, i.e. of the opening becoming larger is also stopped in the space between the two parallel plastic mesh sheets.

We also recognised that during the surgical interventions performed in the recent past it could be determined that there is actually no need for the V-shaped groove on the cylindrical tube member linking the two parallel sheets with an opening in the middle of the device according to the Hungarian patent application referred to above which is designed to accommodate the blood supply arteries for the stoma to be pulled through it in order to provide a suitable blood supply for the stoma. However, it is unfavourable from the point of view that it may promote early falling forward or slipping out following the intestine operation. By forming the cylindrical wall of the tube member channel without the continuous, longitudinal groove this problem may be overcome and if we select the diameter of the tube member in accordance with the diameter of the intestine to be led through it, the risk of the artery or artery bundle providing the blood supply for the extracted stoma possibly becoming blocked and of the stoma dying can be reassuringly eliminated.

Furthermore, we also recognised that the scarred adhesion occurring as detailed above of the tube member made of plastic mesh or woven plastic to the wall of the intestine can be overcome by coating the internal surface of the tube member coming into contact with the wall of the intestine with a coating that does not cause an inflammatory reaction with human tissue, i.e. with the wall of the intestine, which only adheres to the surface of the intestine so that the adhesion between the tube member and the intestine can be relatively easily separated if it becomes necessary. As according to our observations when the two surfaces come into contact the so-called "serosal" surface of the intestine excretes a collagen-like substance that can be separated from the plastic easily without the elements of the intestine wall becoming injured. The coating forms a sufficiently strong bond between the two surfaces - the mesh coated with silicon and the external surface of the intestine wall - so that the internal pressure of the abdominal cavity is unable to pull them apart from each other. However, during a new surgical intervention that possibly becomes necessary the two surfaces can be easily separated from each other. With this solution we can completely eliminate the occurrence of scarred adhesion between the intestine wall and the material of the mesh that occasionally even penetrates to the internal layers of the intestine wall, and obviously the unfavourable consequences deriving from it and detailed above.

The task to be solved with the invention is included in the features contained in claim 1, and the practical embodiments are defined in the dependent claims.

So, on the basis of the above recognitions the set task according to the invention was solved with a device used for the reconstruction of parastomal hernias and/or for the prevention of their development, which has sheets made from plastic compatible with living tissue placed in the abdominal wall running at a distance from each other and connected with a linking-separating element, which linking-separating element is formed by a tube member open at both ends forming a channel to make it possible to pull out a loop of the intestine from the abdominal cavity through the abdominal wall, and at least the tube member is coated with an adhesive plastic, and characteristic of the device is that the fascias and the muscular layer of the abdominal wall in the vicinity of the tube member is fixed to the sheets via fixing elements - favourably passing through all of the fascias and muscle layers.

The sheets of the device according to the invention may be made from a material identical to that of the tube member, but also from a different material, but also from a different pp material, even from solid plastic sheet (foil) as well; this latter solution may be favourable basically from the point of view of cost. It is also possible that the sheets are each made from a different material: for example, dangerfree contact of the device with the abdominal cavity organs is made possible if the internal sheet is made from the aforementioned "Goretex", or from material of this nature: in this case this embedding of the device is technically simpler, however, its price is higher. One component of the material of the tube member or of the sheets may be a material that is absorbed into the body as well.

According to an advantageous embodiment the fixing elements are formed by transfascial sutures passing through the sheets, as well as through the outer and inner fascia, furthermore through the abdominal wall muscle between them, favourably made from monophile polypropylene, non-absorbing thread. With this solution we eliminate the problem detailed in the introduction that scarred bonds may occur between the intestine wall and the connection tube member made from plastic mesh, which may involve the damaging consequences detailed there. Such materials are distributed, for example, by Bondex Kft. under the brand name Medi-sil (EU 82/711/EEC and 93/8/EEC, and USAFD-CFR21,177.2600 classification).

So the application of the device includes that during the operation the fixing is solved with transfascial sutures passing through the sheets, the outer and inner fascias, as well as through the abdominal wall muscle between them, favourably from monophile polypropylene, non-absorbing thread. It is favourable if the transfascial sutures are U-shaped through the internal sheet and also hold the peritonium, and passing through the external sheet they are knotted outside the outer fascia. It is also favourable if the sutures are located at symmetrically allocated positions as compared to the geometrical longitudinal axis of the device.

According to an advantageous embodiment the internal surface of the central linking tube member is a continuous smooth surface. With this solution we eliminate the disadvantage of the previously presented solution that the longitudinal groove used in its cylindrical walled connection member may cause postoperative bowel prolapse an slipping out of the mesentery. However, the continuous, smooth internal surface of the linking member - i.e. not containing a protruding shape or depression, e.g. longitudinal groove - with the correct selection of the diameter of the linking tube member eliminates the risk of the artery or artery bundle providing the blood supply for the stoma possibly becoming blocked and of stoma necrosis.

According to another embodiment at least the tube member of the device is made from plastic mesh and/or woven plastic textile. It is practical to produce the device in various sizes so that it is not necessary to cut the mesh to size, because this may lead to the unravelling of the edges, which weakens the supporting ability of the mesh.

According to an especially favourable embodiment the internal surface of the tube member linking the sheets is coated with a favourably silicon-based material that causes easily releasable adhesion with the surface of the intestine.

It is favourable if the sheets are parallel to each other, and if the geometric longitudinal axis of the tube member passes through the geometric centre point of the sheets; and also if the sheets are circular and produced in various diameters; furthermore, if the device has a circular cylinder cross-section tube member, the diameter of which is selected considering the diameter of the bowel to be led through it. In this respect we note that the diameter of the tube member should be selected so as to prevent the early falling forward, slipping out of the bowel after surgery, however, the fit between the bowel and the tube member should not be so tight so as to have a damaging effect on the blood supply to the bowel. It is practical to manufacture the length of the tube in various lengths due to the differences in the thickness of the abdominal wall of different individuals.

The cross-sectional shape of the tube member is most favourably circular, but other shapes may also be chosen, e.g. elliptical.

During the surgical procedure eliminating the hernia, the hernia sac formed around the opening of the large intestine located in the abdominal wall is dissected; the envelope formed by the peritoneum is left alone from the edge of the stoma aperture; the contents of the hernia and the large intestines are separated and placed back into the abdominal cavity; the musculo-aponeurotic layer surrounded by fascia is tightened; the inner sheet of the medical device is located above the peritoneum, under the musculo-aponeurotic layer, then the large bowel placed back into the abdominal cavity and destined to be the orifice is pulled through the tube member of the medical device above the external surface of the skin, then fixed to the abdominal wall with sutures. A feature of the invention is that following the extraction of the large intestine stoma instrumented through the tube member, the medical device is fixed to the musculo-aponeurotic layer of the abdominal wall favourably with the help of sutures at four points symmetrically arranged as compared to the geometrical longitudinal axis of the device. One of the most important methods of conception of the procedure is that the muscle layer is fixed to the device with transfascial sutures. It is favourable if the transfascial sutures are performed using monophile polypropylene, non-absorbing thread with U-shaped sutures passing through the internal sheet and also holding the peritoneum from the outside, and passing through the external sheet they are knotted outside the outer fascia. The two plastic mesh sheets are fixed to each other with sutures passing through every layer, from the external tendinous abdominal wall to the peritonium.

Favourably the device may be impregnated with silver for the purpose of preventing infection or rejection, based on the known antibacterial effect of silver.

In the following we present the invention in detail on the basis of the attached drawings, which contain an advantageous embodiment of the medical device, and which illustrate the method of its application. In the drawings
Figure 1a. shows an embodiment of the medical device according the invention in perspective view;
Figure 1b. shows the device according to figure 1a. in top view;
Figure 2 shows a perspective view of the medical device according to figure 1 with the large intestine pulled through the tube member and the abdominal wall;
Figure 3 shows an axial cross-section of the device according to figure 2 illustrating its position in the abdominal wall;
Figure 4 shows the device according to the invention in top view also showing the large intestine pulled through its tube member.

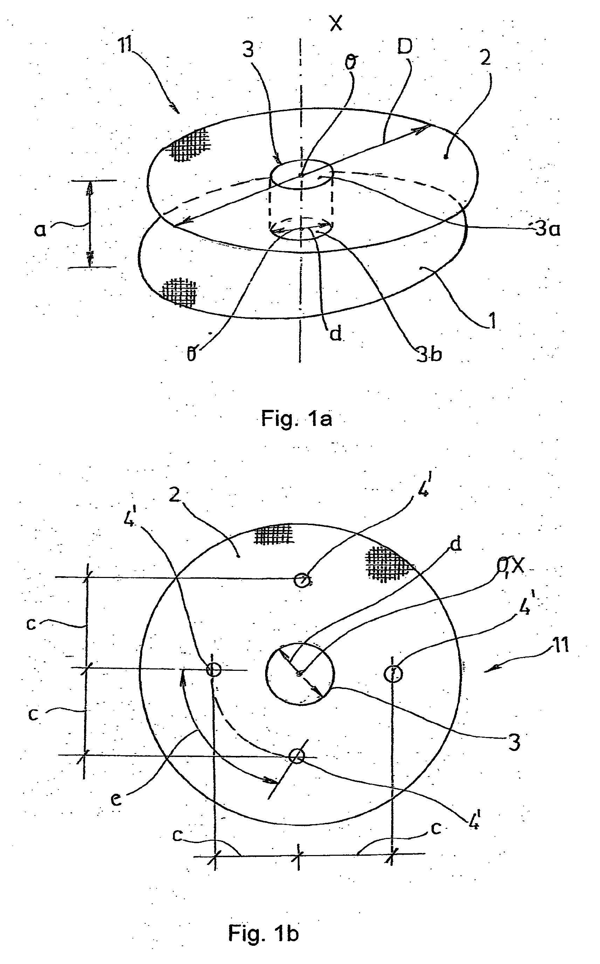

The medical device according to the invention marked with reference number 11 on figure 1a. has an internal sheet 1 an external sheet 2 running in parallel with it at a distance a, and a cylindrical tube member 3 linking these sheets to each other, which is open at both ends, i.e. it has an external opening 3a and an internal opening 3b. The sheets 1, 2 and the tube member 3 together form a relatively rigid unit. The tube member 3 is fixed to the sheets 1, 2 along its end edges, therefore, the openings 3a, 3b fall in the plane of the sheets 1, 2. As it can be easily seen in figure 1 the curved wall of the tube member 3 is continuous and smooth, it is unbroken by any groove or other depressions.
In the case of this embodiment the sheets 1, 2 are circular and identical in size, the geometric axis X passes through their centre point o, which is the longitudinal centre axis of the circular tube member 3. The diameter D of the sheets 1, 2 is favourably 10-12 cm, while the diameter d of the cylindrical tube member 3 may be between 2-3 cm. Both the sheets 1, 2 and the tube member 3 are formed from flexible plastic mesh made from living tissue-friendly plastic mesh, favourably from the aforementioned, commercially available "Goretex", which is known in itself, or from a polypropylene or polypropylene-based material, their thickness and rigidity is selected in accordance with the values usual in the given specialist field. It may be favourable if the sheets 1, 2 are of differing size, for example, the internal sheet has a larger diameter than the external sheet.

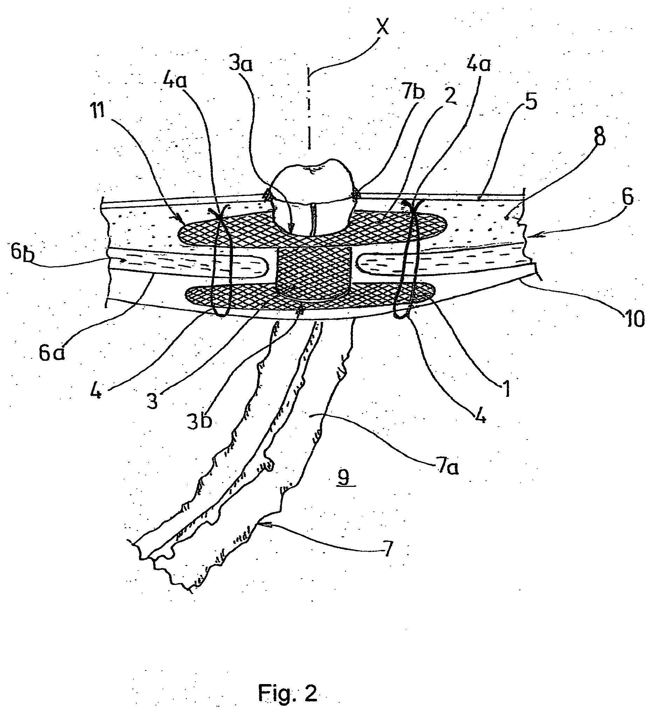

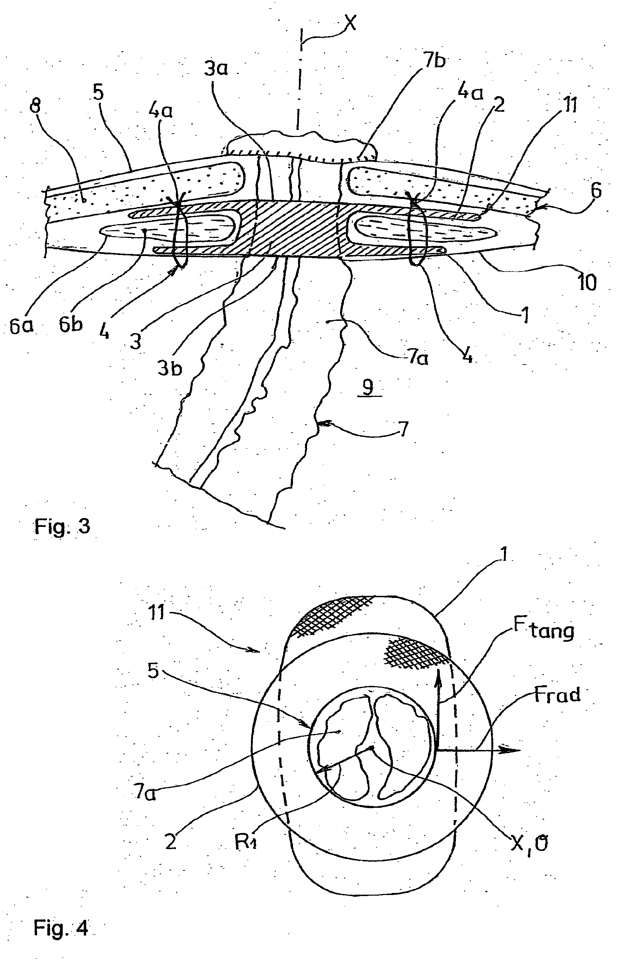

In figures 2 and 3 we have shown the medical device 11 in its entirety built into the abdominal wall 6 delimiting the abdominal cavity 9 from the outside, in the phase of an operation aimed at the reconstruction of a parastomal hernia when the stoma 7a of the large intestine 7 is led out in front of the abdominal wall 6 and its external end 7b is already fixed to the abdominal wall 6 with a suture. On the outside the abdominal wall 6 is delimited by the skin layer 5, under it is the fat layer 8 (subcutaneous fat), under which lies the musculo-aponeurotic abdominal wall layer 6b encompassed by the fascia 6a (membrane covering muscles), which is delimited from below by the peritoneum 10 (abdominal membrane), which separate the abdominal wall 6 from the abdominal cavity 9. We note that if the internal sheet 1 - viewed from the abdominal cavity 9 - is made from "Goretex" it may be positioned inside the peritoneum 10.

According this embodiment of the device 11 according to the invention it has four fixing elements indicated with reference number 4 on figures 2 and 3, which link the device 11 and the musculo-aponeurotic abdominal wall layer 6b of the abdominal wall 6 together, and through this a connection is created between this abdominal wall layer and the device 11 that essentially prevents movement of the device and this abdominal wall layer as compared to each other, together, however, they can turn. In the case of this embodiment the fixing elements 4 are so-called transfascial sutures, which are positioned between the edges of sheets 1 and 2 and the tube member 3, at the same lateral distances c from the centre points o of these sheets indicated on figures 1a and 1b, and at equal lateral distances e from each other measured on a curve, in other words symmetrically around the geometric axis x of the device 11, as we have shown on figure 1b, where the geometric positions of the fixing elements 4 are indicated by points 4'. Obviously the points 4' are also at an equal distance from the edge of the tube member 3. It can be seen well on figures 2 and 3 that the fixing elements 4 are formed by transfascial sutures, which pass through the sheets 1 and 2, the musculo-aponeurotic abdominal wall layer 6b encompassed by the fascia 6a, and the peritoneum 10, under which they are pulled through sheet 2 in a "U" shape. The knots 4a of the fixing elements 4 are established above sheet 1, with the thread passing through it, i.e. they are located here.

As a result of the fixing described above the central tube member of the device 11, through which we pull the large intestine during the course of the operation, fills the primary role of not being able to expand further as a consequence of the forces that are exerted on the edge of the hole passing through the abdominal wall. In practice parastomal hernias only develop as a consequence of these forces, as we have illustrated via figure 4. We note that by eliminating the expansion of the size of the hole we minimise the risk of the development of a hernia.

Illustrating the force effects according to figure 4 - where we have shown the mesh material sheets 1, 2 with different shapes to each other to better show the three-dimensional effect, namely the internal sheet 1 is elliptical and the external sheet 2 is circular - according to Laplace's law the forces exerted on the tube member 3, or on the edge of the hole passing through the abdominal wall are directly related to the radius R¹ of the hole. It has also been shown that the so-called tangential forces F^{tang} exerted on the edge of the hole, besides being dependent on the radius R¹ of the hole 5 or opening, depend on the abdominal cavity pressure P, as well as on the radius R² of the abdomen (not shown on figure 4) according to the following formula:
F^{tang}= F^{rad}R¹ (force exerted on the edge of the stoma opening)
F^{rad}=P×R²/2 (the force exerted on the abdominal wall from the inside)
K¹=2π.R²
K²=2 π.R¹
K¹= the circumference of the abdomen; and
K²= the circumference of the stoma.
(Source: De Ruiter P, Bijnen AB: Successful local repair of paracolostomy hernia with a newly developed prosthetic device. Int J Colorectal Dis 1992; 7:132-34 and De Raet J et al.: Waist circumference is an independent risk factor for the development of parastomal hernia after permanent colostomy. Dis Colon Rectum, 2008. 51 (12): 1806-9).

So fixing the device 11 and the abdominal wall 6 to each other according to the invention reassuringly eliminates the unfavourable effect of the tangential forces and the further expansion of the opening, and through this it minimises the risk of the development of a hernia.

The execution of the parastomal hernia operation with the help of the medical device 11 according to figures 1a-3 takes place in the following way:
The hernia (not illustrated) developed around the opening of the large intestine 7 positioned on the abdominal wall 6 is dissected - in a way known in itself - from an incision made around the opening, the envelope formed by the peritoneum 10 is left from the edge of the hernial orifice. We resolve the adhesions of the content of the hernia, then separating the content of the hernia and the large intestines from each other we place them back into the abdominal cavity 9. We tighten the musculo-aponeurotic layer 6b (not visible in the drawing) surrounded by the fascia 6a with sutures leaving a hole of distance d. We dissect in the peritoneum 10 and the fascia 6a layer, then we insert the medical device 11 with its upper sheet 2 clamped together with its lower sheet 1 with an appropriate clamping instrument above the peritoneum 10 but under the musculo-aponeurotic layer 6b. The external sheet 2 is placed onto the fascia 6a, above the musculo-aponeurotic layer 6b. The device 11 is fixed to the abdominal wall 6 with transfascial sutures described above, establishing by this the fixing elements 4 at 4 positions according to figure 1b (figures 2 and 3). As a result of this fixing operation - in other words with a "U" shaped lower suture with non-absorbing thread from monophile polypropylene usually used in surgical techniques and with the knotting of the thread we have fixed the vicinity of the stoma 7a, with this we have ensured that the environment of the stoma and the mesh move together both on the external and internal surface, and have eliminated the possibility of opposite direction twisting. Besides this with the immediate fixing the risk of the stretching out of the edges of the opening formed in the abdominal wall 6, i.e. the enlarging of the opening in the area between the two sheets is also eliminated. Following this the stoma 7a destined to be the orifice previously placed into the abdominal cavity 9 is pulled though the tube member 3 of the medical device 11, and is raised approx. 2 cm above the external surface of the skin layer 5 of the abdominal wall.

As a result of this operation the supporting layer of the abdominal wall 6, in other words the musculo-aponeurotic layer 6b covered with fascia 6a gets between the two sheets 1, 2 surrounding the edge of the cylindrical tube member 3 at a width of about 2 cm. It is not necessary to take separate measures to fix the large intestine 7, more precisely the pulled through stoma 7a to the cylindrical tube member 3 because due to the very strong adhesive ability and scarring forming feature of the plastic mesh material - in a way known in itself - it reliably prevents omentum or other intestine sections slipping in between the wall of the tube member 3 and the pulled through stoma 7a, however, the problem detailed previously, scarred adhesion between the intestine wall and the mesh material of the tube member, may occur. Due to this, during production the internal surface of the cylindrical tube member 3 connecting the sheets 1, 2, which comes into direct contact with the wall of the intestine, should be favourably coated with a silicon-based material, which does not, or only loosely adheres to the surface of the stoma 7a, which creates easily releasable adhesion between the mesh and the intestine. Such material may be commercially acquired from, for example Bondex Kft. under the brand name of Medi-sil (EU 82/711/EEC and 93/8/EEC and USAFDA-CFR21, I77.2600 classification).

We put a drain in the place of the eliminated hernia sac, then we suture the aforementioned circular skin incision together with the edge of the pulled through stoma 7a; this suturing is indicated with reference number 7b.

The diameter of the stoma 7a of the large intestine 7 to be pulled through varies depending on the anatomical features of the individual, therefore it is favourable to produce the cylindrical tube member 3 with various diameters. Similarly, due to the possible differences in thickness between individuals of the musculo-aponeurotic layer 6b of the abdominal wall 6 it is also favourable to have the product manufactured with the distance a between the sheets 1, 2 at various or at least two different sizes. If we are not performing a hernia reconstruction operation but an operation aimed at preventing the development of a parastomal hernia the hernia dissection is not performed and there is no hernial sac covered by the peritoneum 10; otherwise the other steps of the medical device 11 insertion operation - including the fixing of the device to the abdominal wall - are the same as those detailed above.

If the internal sheet 1 of the device is made from "Goretex" or material of a similar nature, this sheet 1 may be positioned within the peritoneum 10, it may be in direct contact with the organs within the abdominal cavity 9, and may be linked directly to them. Its fixing may take place with the above described transfascial sutures.

The advantageous effects linked to the invention are summarised in the following:
using a simple device, the invention makes possible the reconstruction of parastomal hernias so that their reoccurrence, hernia relapse and the occurrence of septic and other complications can be viewed as being reliably excluded, and the device when preparing the stoma, through the strengthening of the abdominal wall area in the vicinity of the pulled out intestine, provides the possibility for preventing the development of a hernia. An outstanding advantageous feature of the invention is that due to fixing the device and the abdominal wall to each other we have eliminated the risk that occurred in the case of an earlier such solution when due to the twisting movement of the torso the sheets may twist the cylindrical connection tube member, and as the intestine follows this twisting motion, damaging, smaller or larger strictures may occur in the latter. Furthermore, we have eliminated the possibility of the early falling forward or slipping out of the omentum, furthermore, the risk of the scarred adhesion of the material of the plastic mesh to the intestine wall.

Naturally, the invention is not restricted to the embodiments of the medical device detailed above, or to the described method of conception of the procedure, it may be realised in several ways within the sphere of protection defined by the claims.

## Claims

1. Medical device for the reconstruction of parastomal hernias and/or for the prevention of their development, having sheets (1, 2) which are made from plastic compatible with living tissue and which can be placed in the abdominal wall and which are connected via a linking-separating element and which are running separated at a distance (a) from each other, which linking-separating element is formed by a tube member (3) open at both ends forming a channel to make it possible to extract the large intestine out of the abdominal cavity through the abdominal wall and at least the tube member (3) is made from an adhesive plastic suitable for creating an organic connection with live tissue, **characterised by** that the two sheets (1, 2) are connected via fixing elements (4) outside of the tube member (3) for fixing the musculo-aponeurotic layer of the abdominal wall between the sheets (1, 2) in the vicinity of the tube member (3) to the sheets (1, 2) and the tube member (3) has a continuous and smooth wall unbroken by any groove or other depressions.

2. The medical device according to claim 1, **characterised by** that the layer of the abdominal wall is fixed to the sheets (1, 2) via fixing elements (4) passing through all of the fascias and muscle layers.

3. The medical device according to claim 1 or 2, **characterised by** that the fixing elements (4) are formed by transfascial sutures passing through the sheets (1, 2), as well as through the outer and inner fascia, furthermore through the abdominal wall muscle between them, made from monophile polypropylene, non-absorbing thread.

4. The medical device according to claim 3, **characterised by** that the transfascial sutures are U-shaped through the internal sheet (1) and also hold the peritoneum, and passing through the external sheet (2) they are knotted (4a) outside the outer fascia.

5. The medical device according any of claims 1-4, **characterised by** that it has four fixing elements (4) located at symmetrically allocated positions as compared to the geometrical longitudinal axis (x) of the device (11).

6. The medical device according any of claims 1-5, **characterised by** that the internal cylindrical surface of the central linking tube member (3) is a continuous smooth surface.

7. The medical device according any of claims 1-6, **characterised by** that at least the tube member (3) is made from plastic mesh and/or woven plastic textile.

8. The medical device according any of claims 1-7, **characterised by** that the internal surface of the tube member (3) linking the sheets (1, 2) is coated with a silicon-based material that causes easily releasable adhesion with the surface of the intestine.

9. The medical device according any of claims 1-8, **characterised by** that the sheets (1, 2) are parallel to each other, and the geometric longitudinal axis (x) of the tube member (3) passes through the geometric centre point (o) of the sheets (1, 2).

10. The medical device according any of claims 1-9, **characterised by** that the sheets (1, 2) are circular and have the same diameter (D).

11. The medical device according any of claims 1-9, **characterised by** that it has a circular cylinder cross-section tube member (3), the diameter (d) of which is selected in accordance with the diameter of the stoma to be led through it.

## Patentansprüche

1. Eine medizinische Vorrichtung zur Rekonstruktion parastomaler Hernien und/oder zur Verhinderung von deren Entwicklung mit Platten (1, 2), die aus einem mit Lebendgewebe verträglichen Kunststoff hergestellt sind und die in der abdominalen Wand platziert werden können und die über ein Verbindungstrennelement verbunden sind und die in einem Abstand (a) getrennt voneinander verlaufen, welches Verhindungstrennelement durch ein an beiden Enden offenes Rohrelement (3) gebildet ist, das einen Kanal bildet, um es zu ermöglichen, den Dickdarm durch die abdominale Wand aus dem abdominalen Hohlraum herauszuziehen und wenigstens das Rohrelement (3) ist aus einem klebenden Kunststoff hergestellt, der zur Schaffung einer organischen Verbindung mit Lebendgewebe geeignet ist,
dadurch gekennzeichet, dass die zwei Platten (1, 2) über Befestigungselemente (4) außerhalb des Rohrelementes (3) für eine Fixierung der muskulo-aponeurotischen Schicht der abdominalen Wand zwischen den Platten (1, 2) in der Nähe des Rohrelementes (3) an den Platten (1, 2) verbunden sind und das Rohrelement (3) eine kontinuierliche und glatte Wand hat, die nicht durch irgendeine Nut oder andere Vertiefungen unterbrochen ist.

2. Die medizinische Vorrichtung nach Anspruch 1, dadurch gekennzeichnen, dass die Schicht der abdominalen Wand über Befestigungselemente (4), die durch jede der Faszien und Muskelschichten hindurch verlaufen, an den Platten (1, 2) befestigt ist.

3. Die medizinische Vorrichtung nach Anspruch 1 oder 2, **dadurch gekennzeichnet, dass** die Befestigungselemente (4) durch transfasziale Fäden gebildet sind, die durch die Platten (1, 2) hindurch sowie durch die äußere und innere Faszie hindurch, ferner durch den abdominalen Wandmuskel zwischen ihnen hindurch, verlaufen und aus einem nicht absorbierenden monophilen Polypropylenfaden hergestellt sind.

4. Die medizinische Vorrichtung nach Anspruch 3, **dadurch gekennzeichnet, dass** die transfaszialen Fäden U-förmig durch die innere Platte (1) hindurch sind und auch das Bauchfell halten und sie durch die äußere Platte (2) hindurch verlaufend außerhalb der äußeren Faszie verknotet (4a)sind.

5. Die medizinische Vorrichtung nach einem der Ansprüche 1-4, **dadurch gekennzeichnet, dass** sie vier Befestigungselemente (4) hat, die sich an symmetrisch verteilten Positionen verglichen mit der geometrischen Längsachse (x) der Vorrichtung (11) befinden.

6. Die medizinische Vorrichtung nach einem der Ansprüche 1 - 5, **dadurch gekennzeichnet, dass** die innere Zylinderoberfläche von dem zentralen Verbindungsrohrelement (3) eine kontinuierliche glatte Fläche ist.

7. Die medizinische Vorrichtung nach einem der Ansprüche 1 - 6, **dadurch gekennzeichnet, dass** wenigstens das Rohrelement (3) aus einem Kunststoffnetz und/oder einem gewobenen Kunststoffgewebe hergestellt ist.

8. Die medizinische Vorrichtung nach einem der Ansprüche 1-7, **dadurch gekennzeichnet, dass** die innere Oberfläche von dem Rohr-element (3), das die Platten (1, 2) verbindet, mit einem Silicium-basierten Material beschichtet ist, das eine leicht lösbare Haftung mit der Oberfläche Darms bewirkt.

9. Die medizinische Vorrichtung nach einem der Ansprüche 1 - 8, **dadurch gekennzeichnet, dass** die Platten (1, 2) parallel zueinander sind und die geometrische Längsachse (x) von dem Rohrelement (3) durch den geometrischen Mittelpunkt (o) von den Platten (1, 2) hindurch verläuft.

10. Die medizinische Vorrichtung nach einem der Ansprüche 1 - 9, **dadurch gekennzeichnet, dass** die Platten (1, 2) kreisförmig sind und denselben Durchmesser (D) haben.

11. Die medizinische Vorrichtung nach einem der Ansprüche 1 - 9, **dadurch gekennzeichnet, dass** sie ein Rohrelement (3) mit einem kreisförmigen Zylinderquerschnitt hat, wobei dessen Durchmesser (d) in Übereinstimmung mit dem Durchmesser des dort hindurch zu führenden Stoma ausgewählt ist.

## Revendications

1. Dispositif médical pour la reconstruction de hernies parastomales et/ou pour la prévention de leur développement, ayant des feuilles (1, 2) qui sont faites de plastique compatible avec les tissus vivants et qui peuvent être placées dans la paroi abdominale et qui sont reliées via un élément de liaison-séparation et qui courent séparées à une distance (a) l'une de l'autre, lequel élément de liaison-séparation est formé par un élément tubulaire (3) ouvert aux deux extrémités formant un canal pour permettre d'extraire le gros intestin hors de la cavité abdominale à travers la paroi abdominale et au moins l'élément tubulaire (3) est fait d'un plastique adhésif approprié pour créer une connexion organique avec du tissu vivant, **caractérisé en ce que** les deux feuilles (1, 2) sont reliées via des éléments de fixation (4) à l'extérieur de l'élément tubulaire (3) pour fixer la couche musculo-aponévrotique de la paroi abdominale entre les feuilles (1, 2) au voisinage de l'élément tubulaire (3) aux feuilles (1, 2) et l'élément tubulaire (3) a une paroi continue et lisse non interrompue par une quelconque rainure au autres dépressions.

2. Dispositif médical selon la revendication 1, **caractérisé en ce que** la couche de la paroi abdominale est fixé aux feuilles (1, 2) via des éléments de fixation (4) passant à travers toutes les fascias et les couches musculaires.

3. Dispositif médical selon la revendication 1 ou 2, **caractérisé en ce que** les éléments de fixation (4) sont formés par des sutures transaponévrotique passant à travers les feuilles (1, 2), aussi bien qu'a travers le fascia extérieur et intérieur, en outre à travers le muscle de la paroi abdominale entre eux, faites de polypropylène monophile, n'absorbant pas le fil.

4. Dispositif médical selon la revendication 3, **caractérisé en ce que** les sutures transaponévrotiques sont en forme de U à travers la feuille interne (1) et maintiennent également le péritoine, et en passant à travers la feuille externe (2) elles sont nouées (4a) à l'extérieur du fascia extérieur.

5. Dispositif médical selon l'une quelconque des revendications 1 à 4, caractérisé en qu'il a quatre éléments de fixation (4) situés à des positions attribuées de façon symétrique en comparaison à l'axe longitudinal géométrique (x) du dispositif (11).

6. Dispositif médical selon l'une quelconque des revendications 1 à 5, **caractérisé en ce que** la surface cylindrique interne de l'élément tubulaire de liaison centrale (3) est une surface lisse continue.

7. Dispositif médical selon l'une quelconque des revendications 1 à 6, **caractérisé en ce qu'**au moins l'élément tubulaire (3) est fait de maille de plastique et/ou de textile de plastique tissé.

8. Dispositif médical selon l'une quelconque des revendications 1 à 7, **caractérisé en ce que** la surface interne de l'élément tubulaire (3) liant les feuilles (1, 2) est revêtue d'une matière à base de silicone qui provoque une adhérence pouvant être facilement libérée avec la surface de l'intestin.

9. Dispositif médical selon l'une quelconque des revendications 1 à 8, **caractérisé en ce que** les feuilles (1, 2) sont parallèles l'une à l'autre, et l'axe longitudinal géométrique (x) de l'élément tubulaire (3) passe par le point central géométrique (o) des feuilles (1, 2).

10. Dispositif médical selon l'une quelconque des revendications 1 à 9, **caractérisé en ce que** les feuilles (1, 2) sont circulaires et ont le même diamètre (D).

11. Dispositif médical selon l'une quelconque des revendication 1 à 9, **caractérisé en ce qu'**il a un élément tubulaire (3) de section transversale cylindrique circulaire, dont le diamètre (d) est sélectionné selon le diamètre de la stomie à conduire à travers ce dernier.
